# FASCICULE DE BREVET EUROPEEN

(11) **EP 1 838 232 B1**
(45) Date de publication et mention de la délivrance du brevet: **18.06.2008**
(21) Numéro de dépôt: 05850052.1
(22) Date de dépôt: 29.12.2005
(51) Int. Cl.: A61B 17/80, A61B 17/70, F16B 41/00, F16B 25/00

(54) **SYSTEME DE FIXATION DU TYPE PAR VIS IMPERDABLE**
FIXIERSYSTEM MIT HALTESCHRAUBEN
FIXING SYSTEM USING CAPTIVE SCREWS

(30) Priorité: 17.01.2005 FR 0500444
(43) Date de publication de la demande: 03.10.2007
(73) Titulaire: ROUX, Didier, 95270 Asnieres-sur-Oise (FR)
(72) Inventeur: ROUX, Didier, 95270 Asnieres-sur-Oise (FR); RENARD, Xavier, 91430 Vauhallan (FR)
(74) Mandataire: Flavenot, Bernard
(86) Numéro de dépôt international: PCT/FR2005/003297
(87) Numéro de publication internationale: WO 2006/075069

(56) Documents cités:
- FR-A- 2 856 272
- US-A1- 2004 260 306
- US-B1- 6 261 291
- US-B1- 6 322 562

## Description

La présente invention concerne les systèmes de fixation par vissage du type par vis imperdable qui trouvent une application particulièrement avantageuse, mais non exclusivement, pour la fixation d'implants chirurgicaux comme des cotyles, des plaques pour ostéosynthèse, etc.

Il est connu des systèmes de fixation sur un corps de toute nature par vissage d'une vis avec l'interposition, entre la tête de la vis et le corps dans lequel doit être vissée la tige filetée de la vis, une rondelle ou tout autre élément comme une plaque de liaison ou analogue.

Ces systèmes de fixation posent essentiellement deux types de problèmes. L'un de ces problèmes est celui du maintien en place à la fois de la vis et de la plaque ou rondelle lors du vissage de la vis. L'autre est celui de la fiabilité du vissage dans le temps, c'est-à-dire le risque du dévissage de la vis pour toute raison que ce soit, par exemple sous l'action de vibrations ou analogues, et même le risque que la vis ne se détache totalement du corps et se perde.

Ce second problème est particulièrement grave dans le cas de la mise en place d'une plaque pour ostéosynthèse, rachidienne ou autre, car le dévissage de la vis ou des vis peut être très dangereux pour le patient dans le corps duquel ces vis ont été implantées pour maintenir la plaque.

Il existe déjà de nombreux dispositifs qui permettent de réaliser des fixations par vissage au moyen de vis de type imperdable, par exemple celui qui est décrit et illustré dans le US-A-2004/260306. Cependant, tous ces dispositifs sont relativement complexes et donc onéreux.

Aussi, la présente invention a-t-elle pour but de réaliser un système de fixation par vissage qui pallie les inconvénients mentionnés ci-dessus des dispositifs de l'art antérieur, tout en ayant une structure très simple, et qui soit d'une utilisation facile.

Plus précisément, la présente invention a pour objet un système de fixation par vissage, comportant:
d'une part une plaque dans laquelle sont réalisés, en étant sensiblement centrés sur un même axe dit "de fixation" sensiblement perpendiculaire au plan de ladite plaque :
   * un premier orifice de forme générale cylindrique de révolution de profondeur **h** et de section transversale égale à **D**,
   * un second orifice d'une section transversale égale à **d** inférieure à **D**, et
   * un logement de dégagement, lesdits premier et second orifices étant situés de part et d'autre du dit logement de dégagement en y débouchant, la hauteur du dit logement de dégagement prise suivant ledit axe de fixation étant égale à **H** et sa largeur prise perpendiculairement au dit axe de fixation ayant une valeur **L** au moins égale à **D**,
et d'autre part une vis de fixation constituée d'une tige portant un filetage, la section transversale **S** de ladite tige, perpendiculaire à son axe, étant au plus égale à la valeur **d**, et d'une tête d'épaulement fixée à une extrémité de ladite tige, caractérisé en ce que ladite tête d'épaulement comporte une partie périphérique agencée pour être apte à se visser dans une partie latérale (13) du premier orifice (10) bordée par la paroi (11) de ce dit premier orifice , la hauteur **E** de ladite tête d'épaulement prise suivant l'axe de la tige étant au plus égale à la hauteur **H** du logement de dégagement.

D'autres **caractéristiques et** avantages de l'invention apparaîtront au cours de la description suivante donnée en regard des dessins annexés à titre illustratif mais nullement limitatif, dans lesquels :

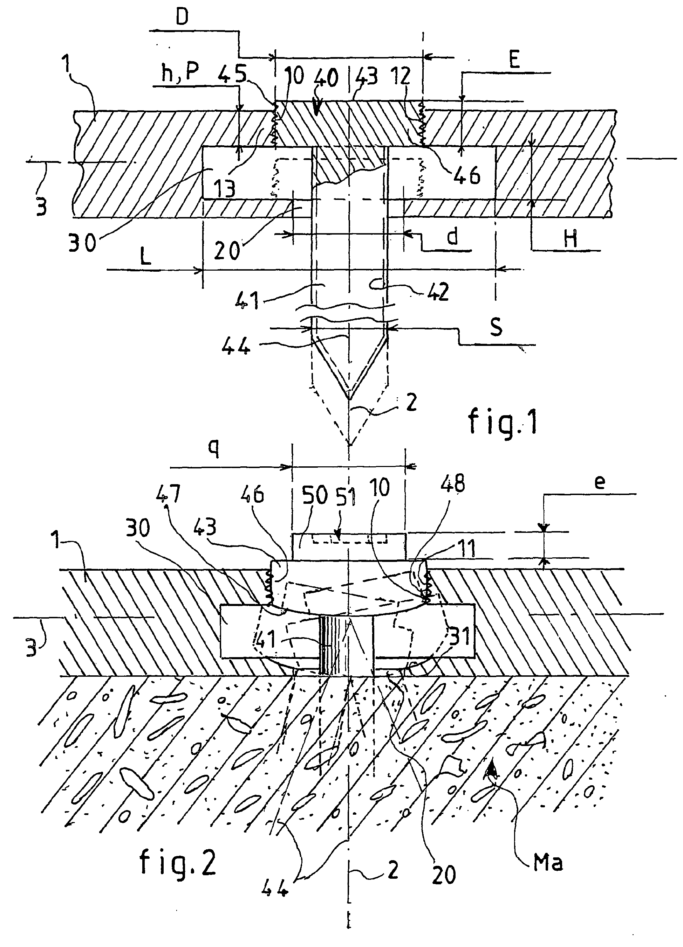

Les figures 1 à 3 représentent respectivement trois modes de réalisation du système de fixation par vissage selon l'invention.

Par référence aux figures 1 à 3 annexées, le système de fixation par vissage comporte une plaque 1 dans laquelle sont réalisés, en étant sensiblement centrés sur un même axe 2 dit "de fixation" sensiblement perpendiculaire au plan 3 de la plaque : un premier orifice 10 de forme générale cylindrique de révolution de profondeur **h** et de section transversale égale à **D**, un second orifice 20 d'une section transversale égale à **d** inférieure à **D**, et un logement de dégagement 30, les premier et second orifices 10, 20 étant situés de part et d'autre du logement de dégagement 30 en y débouchant. La hauteur du logement de dégagement 30 prise suivant l'axe de fixation 2 est égale à **H** et sa largeur prise perpendiculairement à cet axe de fixation a une valeur **L** au moins égale à **D.**

Le système comporte en outre une vis de fixation 40 constituée d'une tige 41 portant un filetage 42, la section transversale **S** de cette tige, perpendiculaire à son axe 44, étant au plus égale à la valeur **d**, et d'une tête d'épaulement 43 fixée à une extrémité de la tige, cette tête d'épaulement comportant une partie périphérique 46 agencée pour être apte à se visser dans une partie latérale 13 du premier orifice 10 bordée par la paroi 11 de cet orifice, la hauteur **E** de la tête d'épaulement prise suivant l'axe 44 de la tige 41 étant au plus égale à la hauteur **H** du logement de dégagement 30.

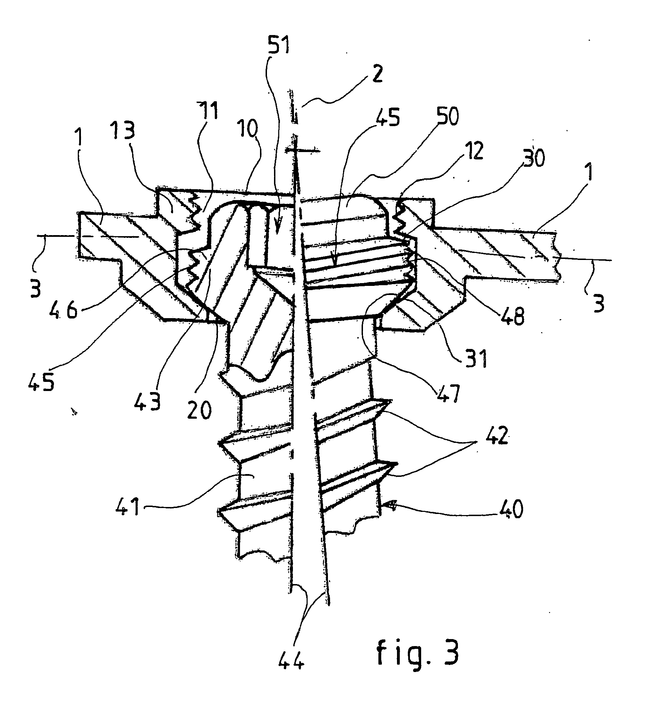

Selon un premier mode de réalisation comme illustré.sur les figures 1 et 3, la paroi 11 du premier orifice 10 comporte un taraudage 12 sur une longueur **P** et la partie périphérique 46 de la tête d'épaulement 43 comporte un filetage 45 complémentaire de ce taraudage 12.

Cependant, dans un autre mode de réalisation possible comme plus particulièrement illustré sur la figure 2, la partie périphérique 46 de la tête d'épaulement 43 et la partie latérale 13 du premier orifice 10 sont respectivement réalisées dans un premier et un second matériaux de dureté différente, l'un des deux éléments "partie périphérique 46 de la tête d'épaulement 43" et "partie latérale 13 du premier orifice 10" comportant un taraudage 12 et étant réalisé dans le matériau le plus dur, l'autre élément étant apte à s'y visser par auto taraudage.

A titre d'exemple, l'un des deux matériaux est du T40, c'est-à-dire du titane pur, et l'autre est du TA6V, c'est-à-dire un alliage de titane.

Il est précisé que le terme "taraudage" utilisé ci-dessus, recouvre aussi bien un taraudage proprement dit pour la partie latérale 13 du premier l'orifice 10, qu'un filetage pour la partie périphérique 46 de la tête d'épaulement 43.

Il est précisé que la partie latérale 13 du premier orifice 10 et la partie périphérique 46 de la tête d'épaulement 43 peuvent être respectivement constituées d'un chemisage de la paroi 11 de l'orifice 10 et/ou d'un manchon situé à la périphérie de la tête d'épaulement 43, les parties restantes de la plaque 1 et de la vis 40 pouvant être dans un même matériau.

Dans ce cas, il est même préférable que ce soit la partie périphérique 46 de la tête d'épaulement 43 qui soit constituée d'un manchon en un matériau comme du T40, et que toute la plaque 1 soit en un matériau plus dur, comme du TA6V. C'est la paroi 11 de l'orifice 10 qui portera un taraudage 12 et c'est la tête d'épaulement qui se vissera sur ce taraudage 12 par auto taraudage, comme dans le cas de la réalisation illustrée schématiquement sur la figure 2.

Selon une réalisation qui est illustrée sur toutes les figures, la valeur **d** est supérieure à la valeur **S**. Dans ce cas, comme plus particulièrement illustré sur les figures 2 et 3, la tige de vis 41 peut prendre différentes positions angulaires par rapport au plan 3 de la plaque 1, du moins dans des limites qui sont généralement suffisantes pour la mise en place notamment de plaques pour ostéosynthèse.

Pour faciliter l'orientation de la tige de vis suivant ces différentes positions angulaires et donner à la tête d'épaulement une bonne assise, la partie de paroi 31 du logement de dégagement 30 bordant l'ouverture par laquelle débouche le second orifice 20 dans ce logement de dégagement présente une forme concave, et la partie 47 de la surface latérale de la tête d'épaulement 43 reliant la tige 41 et la partie 48 de cette surface latérale apte à se visser présente une forme convexe sensiblement complémentaire de la forme concave. Ces formes concave et convexe complémentaires sont avantageusement semi-sphériques ou analogues.

Dans le cas où la plaque 1 est constituée d'une plaque d'ostéosynthèse rachidienne qui doit relier des vertèbres, chaque vis de fixation comporte une tige filetée 41 à filetage osseux auto taraudant.

Dans une réalisation qui peut être avantageuse, notamment pour faciliter la manipulation de la vis de fixation pour l'implanter dans un matériau comme de l'os ou analogue, figure 2 et 3, le système comporte en outre un téton 50 surmontant la tête d'épaulement 43, la section transversale **q** du téton, prise perpendiculairement à l'axe 44 de la tige 41, étant inférieure à la valeur **D**.

Dans le cas d'une application à la mise en place d'une plaque d'ostéosynthèse ou analogue, pour éviter que ce téton 50 ne dépasse de la plaque 1 lorsque la tige 41 est vissée dans un os, la somme de la hauteur **E** de la tête d'épaulement 43 et de la hauteur **e** du téton 50 (figure 2) est au plus égale à la somme de la hauteur **H** du logement de dégagement 30 et de la profondeur **h** du premier orifice 10.

Il est à noter que cette profondeur **h** du premier orifice 10 et la hauteur **P** du taraudage 12 peuvent ne pas être égales, par exemple comme dans le cas où cet orifice 10 est prolongé par un avant trou fraisé de forme tronconique ou cylindrique d'un diamètre supérieur à **D.** Cependant, dans le cas d'un mode de réalisation préféré comme ceux illustrés, ces deux valeurs **h** et **P** sont égales.

Dans le cas d'une application à la mise en place d'une plaque d'ostéosynthèse rachidienne ou autre, il est avantageux que le pas du taraudage 12 du premier orifice 10 soit au plus égal à celui du filetage 42 de la tige filetée 41, et même préférentiellement égal.

## Revendications

1. Système de fixation par vissage, comportant:
d'une part une plaque (1) dans laquelle sont réalisés, en étant sensiblement centrés sur un même axe dit "de fixation" (2) sensiblement perpendiculaire au plan (3) de ladite plaque:
* un premier orifice (10) de forme générale cylindrique de révolution de profondeur **h** et de section transversale égale à **D,**
* un second orifice (20) d'une section transversale égale à d inférieure à **D,** et
* un logement de dégagement (30), lesdits premier et second orifices (10, 20) étant situés de part et d'autre du dit logement de dégagement (30) en y débouchant, la hauteur du dit logement de dégagement (30) prise suivant ledit axe de fixation (2) étant égale à **H** et sa largeur prise perpendiculairement au dit axe de fixation ayant une valeur **L** au moins égale à **D,**
et d'autre part une vis de fixation (40) constituée d'une tige (41) portant un filetage (42), la section transversale S de ladite tige, perpendiculaire à son axe (44), étant au plus égale à la,valeur d, et d'une tête d'épaulement (43) fixée à une extrémité de ladite tige, **caractérisé en ce que** ladite tête d'épaulement comporte une partie périphérique (46) agencée pour être apte à se visser dans une partie latérale (13) du premier orifice (10) bordée par la paroi (11) du dit premier orifice, la hauteur **E** de ladite tête d'épaulement prise suivant l'axe (44) de la tige (41) étant au plus égale à la hauteur **H** du logement de dégagement (30).

2. Système de fixation selon la revendication 1, **caractérisé par le fait que** la paroi (11) du premier orifice (10) comporte un taraudage (12) sur une longueur **P** et que la partie périphérique (46) de ladite tête d'épaulement (43) comporte un filetage (45) complémentaire du dit taraudage (12).

3. Système de fixation selon la revendication 1, **caractérisé par le fait que** la partie périphérique (46) de la tête d'épaulement (43) est réalisée dans un premier matériau et que la partie latérale (13) du dit premier orifice (10) est réalisée en un second matériau, les premier et second matériaux ayant une dureté différente, l'un des deux éléments suivants, "partie périphérique (46) de la tête d'épaulement (43)" et "partie latérale (13) du dit premier orifice (10)" comportant un taraudage (12) et étant réalisé dans le matériau le plus dur, l'autre élément étant apte à s'y visser par auto taraudage.

4. Système de fixation selon la revendication 3, **caractérisé par le fait que** l'un des deux premier et second matériaux est du T40 (titane pur) et que l'autre de ces deux matériaux est du TA6V (alliage de titane).

5. Système de fixation selon l'une des revendications 1 à 4, **caractérisé par le fait que** la valeur **d** est supérieure à la valeur **S**.

6. Système de fixation selon les revendications 1 à 5, **caractérisé par le fait que** ladite tige filetée (41) est à filetage auto taraudant.

7. Système de fixation selon l'une des revendications 1 à 6, **caractérisé par le fait qu'**il comporte un téton (50) surmontant ladite tête d'épaulement (43), la section transversale **q** du dit téton, prise perpendiculairement à l'axe (44) de ladite tige (41), étant inférieure à la valeur **D.**

8. Système de fixation selon la revendication 7, **caractérisé par le fait que** la somme de la hauteur **E** de ladite tête d'épaulement (43) et de la hauteur **e** du dit téton (50) est au plus égale à la somme de la hauteur **H** du logement de dégagement (30) et de la profondeur **h** dudit premier orifice (10).

9. Système de fixation selon l'une des revendications 2 à 8, **caractérisé par le fait que** le pas du taraudage (12) est au plus égal à celui du filetage (42) de la tige filetée (41).

10. Système de fixation selon l'une des revendications 1 à 9, **caractérisé par le fait que** la partie de paroi (31) du dit logement de dégagement (30) bordant l'ouverture par laquelle débouche ledit second orifice (20) dans ce dit logement de dégagement présente une forme concave, et que la partie (47) de la surface latérale de ladite tête d'épaulement (43) reliant la tige (41) et la partie (48) de cette surface latérale apte à se visser présente une forme convexe sensiblement complémentaire de ladite forme concave.

## Claims

1. A screw fastener system, comprising:
firstly a plate (1) having formed therein, substantially centered on a common "fastener" axis (2) substantially perpendicular to the plane (3) of said plate, the following:
* a first orifice (10) of generally circularly cylindrical shape of depth **h** and of cross-section equal to **D,**
* a second orifice (20) of cross-section equal to **d** less than **D**; and
* a clearance housing (30), said first and second orifices (10, 20) being situated on either side of said clearance housing (30) and opening out therein, the height of said clearance housing (30) along said fastener axis (2) being equal to **H**, and its width measured perpendicularly to said fastener axis having a value **L** not less than **D**; and
secondly a fastener screw (40) constituted by a shank (41) carrying a thread (42), the cross-section **S** of said shank, perpendicular to its axis (41), being no greater than the value **d,** and a shouldered head (43) secured to one end of said shank, **characterized by** the fact that said shouldered head comprises a peripheral portion (46) arranged to be suitable for screwing into a side portion (13) of the first orifice (10) bordered by the wall (11) of said first orifice, the height **E** of said shouldered head taken along the axis (44) of the shank (41) being no greater than the height **H** of the clearance housing (30).

2. A fastener system according to claim 1, **characterized by** the facts that the wall (11) of the first orifice (10) includes tapping (12) over a length **P,** and that the peripheral portion (46) of said shouldered head (43) includes a thread (45) complementary to said tapping (12).

3. A fastener system according to claim 1, **characterized by** the facts that the peripheral portion (46) of the shouldered head (43) is made of a first material, and that the side portion (13) of said first orifice (10) is made of a second material, the first and second materials having different hardnesses, one of the following two elements constituted by the "peripheral portion (46) of the shouldered head (43)" and by the "side portion (13) of said first orifice (10) having tapping 12" being made of the harder material, the other element being suitable for self-tapping screw engagement therewith.

4. A fastener system according to claim 3, **characterized by** the fact that one of the first and second materials is T40 (pure titanium) and the other of the two materials is TA6V (titanium alloy).

5. A fastener system according to any one of claims 1 to 4, **characterized by** the fact that the value **d** is greater than the value **S.**

6. A fastener system according to any one of claims 1 to 5, **characterized by** the fact that said threaded shank (41) has a self-tapping thread.

7. A fastener system according to any one of claims 1 to 6, **characterized by** the fact that it includes a stud (50) surmounting said shouldered head (43), the cross-section **q** of said stud, taken perpendicularly to the axis (44) of said shank (41), being less than the value **D.**

8. A fastener system according to claim 7, **characterized by** the fact that the sum of the height **E** of said shouldered head (43) plus the height **e** of said stud (50) is no greater than the sum of the height **H** of the clearance housing (30) plus the depth **h** of said first orifice (10).

9. A fastener system according to any one of claims 2 to 8, **characterized by** the fact that the pitch of the tapping (12) is no greater than that of the thread (42) of the threaded shank (41).

10. A fastener system according to any one of claims 1 to 9, **characterized by** the facts that the wall portion (31) of said clearance housing (30) bordering the opening whereby said second orifice (20) opens out into said clearance housing presents a concave shape, and that the portion (47) of the side surface of said shouldered head (43) connecting the shank (41) to the portion (48) of said side surface that is suitable for screw fastening presents a convex shape that is substantially complementary to said concave shape.

## Patentansprüche

1. System zur Schraubbefestigung, das umfasst:
einerseits eine Platte (1), in der im Wesentlichen zentriert auf dieselbe so genannte "Befestigungs"-Achse (2), die zu der Ebene (3) der Platte im Wesentlichen senkrecht ist, verwirklicht sind:
* eine erste Öffnung (10) mit einer rotationssymmetrischen zylindrischen allgemeinen Form mit Tiefe h und einem Durchmesser, der gleich D ist,
* eine zweite Öffnung (20) mit einem Querschnitt, der gleich d kleiner als D ist, und
* einen Löseaufnahmesitz (30), wobei sich die erste bzw. die zweite Öffnung (10, 20) beiderseits des Löseaufnahmesitzes (30) befinden und in ihn münden, wobei die Höhe des Löseaufnahmesitzes (30) längs der Befestigungsachse (2) gleich H ist und seine Breite senkrecht zu der Befestigungsachse einen Wert L hat, der wenigstens gleich D ist,
und andererseits eine Befestigungsschraube (40), die durch einen ein Außengewinde (42) aufweisenden Stift (41), dessen Durchmesser S senkrecht zu seiner Achse (44) höchstens gleich dem Wert d ist, und aus einem Anschlagkopf (43), der an einem Ende des Stifts befestigt ist, gebildet ist, **dadurch gekennzeichnet, dass** der Anschlagkopf einen Umfangsabschnitt (46) aufweist, der dazu ausgelegt ist, in einen seitlichen Abschnitt (13) der ersten Öffnung (10) geschraubt zu werden, der durch die Wand (11) der ersten Öffnung begrenzt ist, wobei die Höhe E des Anschlagkopfs längs der Achse (44) des Stifts (41) höchstens gleich der Höhe H des Löseaufnahmesitzes (30) ist.

2. Befestigungssystem nach Anspruch 1, **dadurch gekennzeichnet, dass** die Wand (11) der ersten Öffnung (10) auf einer Länge P ein Innengewinde (12) besitzt und dass der Umfangsabschnitt (46) des Anschlagkopfs (43) ein zu dem Innengewinde (12) komplementäres Außengewinde (45) aufweist.

3. Befestigungssystem nach Anspruch 1, **dadurch gekennzeichnet, dass** der Umfangsabschnitt (46) des Anschlagkopfes (43) aus einem ersten Material verwirklicht ist und dass der seitliche Abschnitt (13) der ersten Öffnung (10) aus einem zweiten Material verwirklicht ist, wobei das erste und das zweite Material eine unterschiedliche Härte haben, wobei eines der zwei folgenden Elemente, nämlich der "Umfangsabschnitt (46) des Anschlagkopfes (43)" und der "seitliche Abschnitt (13) der ersten Öffnung (10)" ein Gewinde (12) besitzt und aus dem härteren Material verwirklicht ist, während das jeweils andere Element mit dem ersten Element durch Selbstschneiden eines Gewindes verschraubt werden kann.

4. Befestigungssystem nach Anspruch 3, **dadurch gekennzeichnet, dass** entweder das erste oder das zweite Material T40 (reines Titan) ist und das jeweils andere Material TA6V (Titanlegierung) ist.

5. Befestigungssystem nach einem der Ansprüche 1 bis 4, **dadurch gekennzeichnet, dass** der Wert d größer als der Wert S ist.

6. Befestigungssystem nach den Ansprüchen 1 bis 5, **dadurch gekennzeichnet, dass** der Stift (41) mit Außengewinde ein selbstschneidendes Außengewinde hat.

7. Befestigungssystem nach einem der Ansprüche 1 bis 6, **dadurch gekennzeichnet, dass** es einen Zapfen (50) umfasst, der über den Anschlagkopf (43) vorsteht, wobei der Durchmesser g des Zapfens senkrecht zur Achse (44) des Stifts (41) kleiner als der Wert D ist.

8. Befestigungssystem nach Anspruch 7, **dadurch gekennzeichnet, dass** die Summe aus der Höhe E des Anschlagkopfes (43) und der Höhe e des Zapfens (50) höchstens gleich der Summe aus der Höhe H des Löseaufnahmesitzes (30) und aus der Tiefe h der ersten Öffnung (10) ist.

9. Befestigungssystem nach einem der Ansprüche 2 bis 8, **dadurch gekennzeichnet, dass** die Steigung des Innengewindes (12) höchstens gleich jener des Außengewindes (42) des Gewindestifts (41) ist.

10. Befestigungssystem nach einem der Ansprüche 1 bis 9, **dadurch gekennzeichnet, dass** der Wandabschnitt (31) des Löseaufnahmesitzes (30), der die Öffnung begrenzt, durch die die zweite Öffnung (20) in diesen Löse-Aufnahmesitz mündet, eine konkave Form aufweist und dass der Abschnitt (47) der seitlichen Oberfläche des Anschlagkopfes (43), der den Stift (41) und den Abschnitt (48) dieser seitlichen Oberfläche, der eingeschraubt werden kann, verbindet, eine zu der konkaven Form im Wesentlichen komplementäre konvexe Form aufweist.
